# EUROPÄISCHE PATENTSCHRIFT

(11) **EP 1 115 683 B1**
(45) Veröffentlichungstag und Bekanntmachung des Hinweises auf die Patenterteilung: **26.11.2003**
(21) Anmeldenummer: 99947356.4
(22) Anmeldetag: 15.09.1999
(51) Int. Cl.: C07C 51/08, C07C 57/30

(54) **VERFAHREN ZUR DRUCKLOSEN HERSTELLUNG VON ALPHA, ALPHA-DIMETHYLPHENYLESSIGSÄURE AUS ALPHA, ALPHA-DIMETHYLBENZYLCYANID**
METHOD FOR PRESSURELESS PRODUCTION OF ALPHA,ALPHA-DIMETHYLPHENYL ACETIC ACID FROM ALPHA,ALPHA-DIMETHYL BENZYL CYANIDE
PROCEDE DE PRODUCTION SANS PRESSION D'ACIDE ALPHA,ALPHA-DIMETHYLPHENYLACETIQUE A PARTIR DE ALPHA,ALPHA-DIMETHYLBENZYLCYANURE

(30) Priorität: 26.09.1998 DE 19844225
(43) Veröffentlichungstag der Anmeldung: 18.07.2001
(73) Patentinhaber: Aventis Pharma Deutschland GmbH, 65929 Frankfurt am Main (DE)
(72) Erfinder: KORB, Gerhard, D-63512 Hainburg (DE); FLEMMING, Hans-Wolfram, D-61250 Usingen (DE); LEHNERT, Rudolf, D-55122 Mainz (DE); RYBCZYNSKI, Wolfgang, D-65597 Hünfelden-Ohren (DE)
(86) Internationale Anmeldenummer: EP9906829
(87) Internationale Veröffentlichungsnummer: WO00018715

(56) Entgegenhaltungen:
- BUCKLE D R ET AL: "Synthesis and antiallergic activity of 2-hydroxy-3-nitro-1,4-naphthoquinones" JOURNAL OF MEDICINAL CHEMISTRY, Bd. 20, Nr. 8, August 1977 (1977-08), Seiten 1059-64, XP002128782 in der Anmeldung erwähnt
- CHEMICAL ABSTRACTS, vol. 64, no. 12, 6. Juni 1966 (1966-06-06) Columbus, Ohio, US; MAKOSZA M ET AL: "Reactions of organic anions. III. Synthesis of phenyldialkylacetonitriles" Spalte 17475c; XP002128783 & ROCZNIKI CHEM., Bd. 39, Nr. 11, 1965, Seiten 1595-601,

## Beschreibung

Die Herstellung der α,α-Dimethylphenylessigsäure aus α,α-Dimethylbenzylcyanid ist bekannt. So wird die Herstellung im Journal of Medical Chemistry, 1977, Vol. 20, No. 8, Seite 1063, beschrieben. Dabei wird 2-Phenyl-2-methylpropion-säurenitril (α,α-Dimethylbenzylcyanid) in einer Lösung von KOH (5 facher molarer Überschuß) und Methanol in einem Autoklaven bei 140 bis 150 °C über20 Stunden unter Druck gerührt, anschließend wird das Reaktionsgemisch unter verminderten Druck aufkonzentriert, mit Wasser verdünnt und angesäuert. Die als weißes Kristallisat erhaltene α,α-Dimethylphenylessigsäure wird anschließend durch einen aufwendigen Reinigungsprozess - Filtration und Umkristallisation aus Ethanol - gereinigt. Dabei wird eine α,α-Dimethylphenylessigsäure mit einem Schmelzpunkt von 80 °C erhalten. Die theoretische Ausbeute beträgt 90 %. M. Makosza et al. beschreiben bei der Reaktion von organischen Anionen auch die Synthese von Phenyldialkylacetonitrilen (Roczniki Chem., (39(11), 1965, Seiten 595-601).

Eigene Versuche, bei denen 2-Phenyl-2- methylpropion-säurenitril (α,α-Dimethylbenzylcyanid) in Gemischen aus n-Butanol, Wasser und KOH (3 fach molarer Überschuß) bei etwa 125 °C über 22 Stunden umgesetzt wurde, führten ebenfalls nicht zu einer vollständigen Verseifung des eingesetzten Nitrils. Ferner wurde noch die entsprechende Amid-Zwischenstufe in Mengen von 2 % gefunden. Die Abreicherung dieser Zwischenstufe würde einen zusätzlichen Reinigungsschritt notwendig machen, um eine α,α-Dimethylphenylessigsäure mit großer Reinheit zu erhalten.

Eigene Versuche in Gemischen aus n-Butanol, Wasser und Lithiumhydroxid zeigten nach 4 stündiger Verseifungszeit bei etwa 100 °C (Rückfluß) lediglich die Bildung von etwa 1 % der Amid Zwischenstufe an. Eine Verseifung bis zur Carbonsäurebildung konnte nicht detektiert werden.

Weitere eigene Versuche in 50 %iger wässriger Kali- oder Natronlauge (3 fach molarer Überschuß) bei 125 °C unter kräftigem Rühren zeigten nach 20 Stunden Reaktionszeit folgendes Analysenergebnisse:

| | | | |
|---|---|---|---|
| Kalilauge | Nitril 0,4 % | Amid 51 % | Carbonsäure 47 % |
| Natronlauge | Nitril 62 % | Amid 34 % | Carbonsäure 2,7 % |

Die Verseifungsreaktion mit der stärkeren Base Kaliumhydroxid verlief viel schneller ab als mit der schwächeren Base. Daher ergaben sich folgende Verseifungsgeschwindigkeiten: LiOH < NaOH < KOH

Es wurde nun gefunden, daß in einem Gemisch aus einem C₄-oder C₅-Alkohol, Wasser und NaOH das Nitril bis zur Carbonsäure in kürzester Zeit unter Normaldruck verseift wird.

Die Erfindung bezweckt daher durch Modifizierung der Verfahrensbedingungen eine α,α-Dimethylphenylessigsäure in hohen Ausbeuten zur Verfügung zu stellen, die im wesentlichen frei von 2-Phenyl-2- methylpropionsäurenitril (α,α-Dimethylbenzyl-cyanid) und 2-Phenyl-2-methylpropionsäureamid ist.

Die Aufgabe wird dadurch gelöst, daß die Umsetzung von α,α-Dimethylbenzyl-cyanid in Gegenwart von Natriumhydroxid, Wasser und einem C₄-oder C₅-Alkohol bei Temperaturen über 100 °C durchgeführt wird und anschließend die α,α-Dimethylphenylessigsäure durch Ansäuern gewonnen wird. Zusätzliche Reinigungsschritte sind nicht mehr notwendig.

Die Erfindung betrifft daher ein Verfahren zur Gewinnung der Verbindung der Formel I 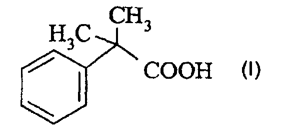 das dadurch gekennzeichnet ist, daß man eine Verbindung der Formel II 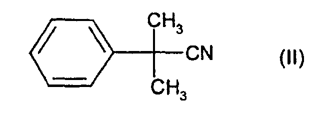 in Anwesenheit von Wasser, Natriumhydroxid und einem C₄-Alkohol oder C₅-Alkohol bei Temperaturen von mehr als 100°C zum entsprechenden Carbonsäuresalz umsetzt und anschließend die Verbindung der Formel I durch Zugabe einer Säure gewinnt.

Bei der Herstellung der Verbindung der Formel I geht man so vor, daß zunächst der C₄oder C₅-Alkohol, Wasser, das Natriumhydroxid und die Verbindung der Formel II (α,α-Dimethylbenzylcyanid) gemischt und auf mehr als 100° unter Rühren erhitzt wird. Es kann auch eine Mischung von C₄- und C₅-Alkohol eingesetzt werden.

Nach einer entsprechenden Reaktionszeit wird das Reaktionsgemisch abgekühlt und mit einer Säure wird α,α-Dimethylphenylessigsäure ausgefällt. Die Isolierung der α,α-Dimethylphenylessigsäure erfolgt beispielsweise durch Kristallisation oder Extraktion. Die Kristallisation wird durch Kühlen der Suspension oder weiteren Verdampfen der Lösemittel gefördert. Die Extraktion erfolgt durch die Zugabe von organischen Lösemitteln zu der α,α-Dimethylphenylessigsäure, beispielsweise Toluol.

Unter dem Begriff C₄- und C₅-Alkohol werden beispielsweise n-Butanol, 2-Methyl-1-propanol, n-Pentanol, 2-Pentanol, 3-Pentanol, 2-Methyl-2-butanol, 3-Methyl-2-butanol, 2-Methyl-3-butanol, 3-Methyl-1-butanol oder 2-Methyl-1-butanol verstanden.

Unter dem Begriff Natriumhydroxid versteht man Ätznatron in fester Form oder in Form von Laugen unterschiedlicher Konzentration. Das in der Lauge vorhandene Wasser geht dann bei der Bereitung der Verseifungsmischung in die Berechnung mit ein.

Geeignete Säuren sind beispielsweise Salzsäure, Schwefelsäure, Salpetersäure oder Phosphorsäure oder Mischungen der Säuren.

Vorzugsweise verwendet man für die Verseifungsreaktion auf 100 Mol der Verbindung der Formel II 120 Mol bis 250 Mol Wasser und 150 Mol bis 300 Mol Natriumhydroxid.

Die verwendete C₄- oder C₅-Alkoholmenge beträgt im allgemeinen von 0,5 kg bis 1,5 kg pro kg der Verbindung der Formel II, bevorzugt von 0,6 kg bis 1,0 kg.

Die Reaktionszeit liegt im allgemeinen von 2 bis 6 Stunden, je nach Zusammensetzung des Gemisches und des gewählten Temperaturbereiches.

Die Reaktionstemperatur beträgt je nach verwendetem C₄- oder C₅-Alkohol von mehr als 100 °C bis 140 °C, bevorzugt von 110 °C bis 130 °C.

Das Nebenprodukt 2-Phenyl-2-methylpropionsäureamid liegt in der isolierten α,α-Dimethylphenylessigsäure mit weniger als 0,1 % bezogen auf die Verbindung der Formel I vor. Der Restgehalt am Ausgangssubstrat der Verbindung der Formel II (α,α-Dimethylbenzylcyanid) konnte in der isolierten α,α-Dimethylphenylessigsäure nicht mehr nachgewiesen werden.

Die Ausgangssubstanzen für die erfindungsgemäße Verseifungsreaktion sind nach literaturbekannten Verfahren herstellbar.
Das Verfahrensprodukt eignet sich für die Herstellung einer Vielzahl von Folgeprodukten, z.B. für die Herstellung von antiallergisch wirkenden Arzneimitteln wie 4-[4-[4-(Hydroxydiphenyl)-1-piperidinyl]-1-hydroxybutyl]-α,α-dimethylphenyl-essigsäure (US 4 254 129).

Vorteilhaft sind die sehr kurzen Reaktionszeiten, der Wegfall von zusätzlichen Reinigungsschritten, die hohen Ausbeuten und die hohe Reinheit des hergestellten Produkts. Vorteil des erfindungsgemäßen Verfahrens ist die im wesentlichen vollständige Umsetzung zur Verbindung der Formel I und ein Gesamtnebenproduktgehalt von weniger als 0,1 %.

### Beispiel 1

Herstellung von α,α-Dimethylphenylessigsäure

In einer Rührapparatur legte man 150 g n-Butanol, 65 g Wasser, 150 g Ätznatron und 221 g Dimethylbenzylcyanid (etwa 98,2 %ig) vor. Das Gemisch wurde unter guter Rührung auf 120 °C bis 126 °C hochgeheizt und bei dieser Temperatur etwa 6 Stunden nachreagieren gelassen. Anschließend wurden 250 ml Wasser zugegeben und das n-Butanol destillativ aus dem Reaktionsgemisch entfernt. Nach Zugabe von weiteren 500 ml Wasser wurde das Gemisch auf 30 °C bis 50 °C abgekühlt. Durch Zugabe von etwa 433 g Salzsäure, etwa 30 %ig, wurde die freie Dimethylphenylessigsäure ausgefällt. Diese konnte entweder isoliert oder aus der Kristallsuspension extrahiert werden. Bei der Isolierung wurde die Suspension weiter abgekühlt, nach Beendigung des Kristallisationsprozesses abgesaugt und mit kaltem Wasser chloridionenfrei gewaschen. Man erhielt nach dem Trocknen im Vakuumtrockenschrank 238,4 g Ausbeute (97,3 % d. Th.). Der Gehalt an Dimethylphenylessigsäure betrug im HPLC mehr als 99,9 % (Amidgehalt weniger als 0,1 %).

Der Schmelzpunkt der so hergestellten α,α-Dimethylphenylessigsäure betrug als Minimum der aufgezeichneten Differentialscanningkalometriemethode 81,5 °C.

### Beispiel 2

Alternativ kann die α,α-Dimethylphenylessigsäure mit einem organischen Lösemittel (z. B. Toluol) extrahiert werden. Dabei wird die gebildete α,α-Dimethylphenylessigsäure zu mehr als 99 % in die organische Phase überführt; die wäßrige Phase wird abgetrennt. Die organische Phase kann dann unmittelbar für Folgereaktionen (z. B. Veresterung) eingesetzt werden.

### HPLC-Bestimmung

### Probenvorbereitung

10 mg Substanz in 10 ml einer Mischung von Acetonitril und Wasser (4:6) lösen.

| | |
|---|---|
| Säule | 0,15 m Länge |
| | 4,6 mm Durchmesser |
| Stationäre Phase | SB-Phenyl auf Kieselgel, 5 µm, Zorbax ® |
| Mobile Phase | 30 % Acetonitril |
| | 70 % Phosphatpuffer, pH.2,5 |
| Injektionsvolumen | 10 µl |
| Fluß | 1 ml/min |
| Detektion | UV, 210 nm |
| Laufzeit | 30 min |

## Patentansprüche

1. Verfahren zur Gewinnung der Verbindung der Formel I, 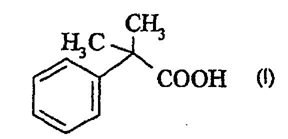 aus einer Verbindung der Formel II 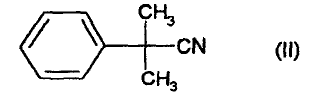 **dadurch gekennzeichnet , daß** man (II)
in Anwesenheit von Wasser, Natriumhydroxid und einem C₄-Alkohol und/oder C₅-Alkohol bei Temperaturen von mehr als 100 °C zum entsprechenden Carbonsäuresalz umsetzt und anschließend die Verbindung der Formel I durch Zugabe einer Säure gewinnt.

2. Verfahren gemäß Anspruch 1, **dadurch gekennzeichnet, dass** man als C₄- oder C₅-Alkohol eine Verbindung aus der Gruppe n-Butanol, 2-Methyl-1-propanol, n-Pentanol, 2-Pentanol, 3-Pentanol, 2-Methyl-2-butanol, 3-Methyl-2-butanol, 2-Methyl-3-butanol, 3-Methyl-1-butanol oder 2-Methyl-1-butanol einsetzt.

3. Verfahren gemäß Anspruch 2, **dadurch gekennzeichnet, dass** man als C₄- oder C₅-Alkohol n-Butanol einsetzt.

4. Verfahren gemäß Anspruch 1 oder 2, **dadurch gekennzeichnet, dass** man eine Mischung von C₄- oder C₅-Alkohol einsetzt.

5. Verfahren nach einem oder mehreren der Ansprüche 1 bis 4, **dadurch gekennzeichnet, dass** man das Natriumhydroxid in Form eines Feststoffs oder als wässrige Lösung einsetzt.

6. Verfahren nach einem oder mehreren der Ansprüche 1 bis 5, **dadurch gekennzeichnet, dass** man als Säure Salzsäure, Schwefelsäure, Salpetersäure oder Phosphorsäure oder Mischungen der Säuren einsetzt.

7. Verfahren gemäß Anspruch 6, **dadurch gekennzeichnet, dass** man als Säure Salzsäure einsetzt.

8. Verfahren nach einem oder mehreren der Ansprüche 1 bis 7, **dadurch gekennzeichnet, dass** man auf 100 Mol der Verbindung der Formel II von 120 Mol bis 250 Mol Wasser und von 150 Mol bis 300 Mol Natriumhydroxid einsetzt.

9. Verfahren nach einem oder mehreren der Ansprüche 1 bis 8, **dadurch gekennzeichnet, dass** man auf 1 kg der Verbindung der Formel II von 0,5 kg bis 1,5 kg des C₄- und/oder C₅-Alkohols einsetzt.

10. Verfahren gemäß Anspruch 9, **dadurch gekennzeichnet, dass** man auf 1 kg der Verbindung der Formel II von 0,6 kg bis 1,0 kg des C₄- und/oder C₅-Alkohols einsetzt.

11. Verfahren nach einem oder mehreren der Ansprüche 1 bis 10, **dadurch gekennzeichnet, dass** man die Umsetzung der Verbindung der Formel II bei einer Temperatur von mehr als 100 °C bis 140 °C durchführt.

12. Verfahren gemäß Anspruch 11, **dadurch gekennzeichnet, dass** man die Umsetzung der Verbindung der Formel II bei einer Temperatur von 110 °C bis 130 °C durchführt.

## Claims

1. A process for obtaining the compound of the formula I 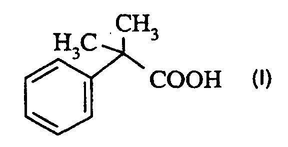 from a compound of the formula II 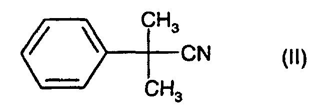 which comprises reacting (II)
in the presence of water, sodium hydroxide and a C₄-alcohol and/or C₅-alcohol at temperatures of more than 100°C to give the corresponding carboxylic acid salt and then obtaining the compound of the formula I by addition of an acid.

2. The process as claimed in claim 1, wherein a compound from the group consisting of n-butanol, 2-methyl-1-propanol, n-pentanol, 2-pentanol, 3-pentanol, 2-methyl-2-butanol, 3-methyl-2-butanol, 2-methyl-3-butanol, 3-methyl-1-butanol and 2-methyl-1-butanol is employed as the C₄- or C₅-alcohol.

3. The process as claimed in claim 2, wherein n-butanol, is employed as the C₄- or C₅-alcohol.

4. The process as claimed in claim 1 or 2, wherein a mixture of C₄- and C₅-alcohol is employed.

5. The process as claimed in one or more of claims 1 to 4, wherein the sodium hydroxide is employed in the form of a solid or as an aqueous solution.

6. The process as claimed in one or more of claims 1 to 5, wherein hydrochloric acid, sulfuric acid, nitric acid or phosphoric acid or a mixture of the acids is employed as the acid.

7. The process as claimed in claim 6, wherein hydrochloric acid is employed as the acid.

8. The process as claimed in one or more of claims 1 to 7, wherein 120 mol to 250 mol of water and 150 mol to 300 mol of sodium hydroxide are employed per 100 mol of the compound of the formula II.

9. The process as claimed in one or more of claims 1 to 8, wherein 0.5 kg to 1.5 kg of the C₄- and/or C₅-alcohol are employed per 1 kg of the compound of the formula II.

10. The process as claimed in claim 9, wherein 0.6 kg to 1.0 kg of the C₄- and/or C₅-alcohol are employed per 1 kg of the compound of the formula II.

11. The process as claimed in one or more of claims 1 to 10, wherein the reaction of the compound of the formula II is carried out at a temperature of more than 100°C to 140°C.

12. The process as claimed in claim 11, wherein the reaction of the compound of the formula II is carried out at a temperature of 110°C to 130°C.

## Revendications

1. Procédé pour l'obtention du composé de formule I 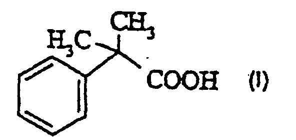 à partir d'un composé de formule II 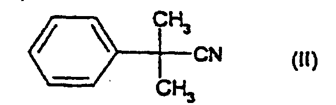 **caractérisé en ce qu'**on fait réagir (II) en présence d'eau, d'hydroxyde de sodium et d'un alcool en C₄ et/ou d'alcool en C₅, à une température supérieure à 100°C pour obtenir le sel d'acide carboxylique correspondant et ensuite on obtient le composé de formule I par ajout d'un acide.

2. Procédé selon la revendication 1, **caractérisé en ce qu'**on utilise comme alcool en C₄ ou en C₅ un composé pris dans le groupe comprenant le n-butanol, le 2-méthyl-1-propanol, le n-pentanol, le 2-pentanol, le 3-pentanol, le 2-méthyl-2-butanol, le 3-méthyl-2-butanol, le 2-méthyl-3-butanol, le 3-méthyl-1-butanol ou le 2-méthyl-1-butanol.

3. Procédé selon la revendication 2, **caractérisé en ce qu'**on utilise le n-butanol en tant qu' alcool en C₄ ou en C₅.

4. Procédé sleon la revendication 1 ou 2, **caractérisé en ce qu'**on utilise un mélange d'alcools en C₄ ou C₅.

5. Procédé selon une ou plusieurs des revendications 1 à 4, **caractérisé en ce qu'**on utilise l'hydroxyde de sodium sous forme d'une matière solide ou sous forme de solution aqueuse.

6. Procédé selon une ou plusieurs des revendications 1 à 5, **caractérisé en ce qu'**on utilise en tant qu'acide l'acide chlorhydrique, l'acide sulfurique, l'acide nitrique ou l'acide phosphorique ou des mélanges de ces acides.

7. Procédé selon la revendication 6, **caractérisé en ce qu'**on utilise en tant qu'acide l'acide chlorhydrique.

8. Procédé selon une ou plusieurs des revendications 1 à 7, **caractérisé en ce qu'**on utilise pour 100 moles d'eau du composé de formule II de 120 moles à 250 moles et de 150 moles à 300 moles d'hydroxyde de sodium.

9. Procédé selon une ou plusieurs des revendications 1 à 8, **caractérisé en ce qu'**on utilise pour 1 kg du composé de formule II de 0,5 kg à 1, 5 kg de l'alcool en C₄ et/ou C₅.

10. Procédé selon la revendication 9, **caractérisé en ce qu'**on utilise pour 1 kg du composé de formule II de 0,6 kg à 1,0 kg de l' alcool en C, et/ou C₅.

11. Procédé selon une ou plusieurs des revendications 1 à 10, **caractérisé en ce qu'**on met en oeuvre la réaction du composé de formule II à des températures supérieures à un intervalle de 100°C à 140°C.

12. Procédé selon la revendication 11, **caractérisé en ce qu'**on met en oeuvre la réaction du composé de formule II à une température de 110 à 130°C.
